# EUROPÄISCHE PATENTSCHRIFT

(11) **EP 0 984 725 B1**
(45) Veröffentlichungstag und Bekanntmachung des Hinweises auf die Patenterteilung: **07.04.2004**
(21) Anmeldenummer: 98924191.4
(22) Anmeldetag: 23.04.1998
(51) Int. Cl.: A61B 17/32, A61B 17/22

(54) **HÄMORRHOIDENRESEKTIONSINSTRUMENT**
INSTRUMENT FOR RESECTIONING HAEMORRHOIDS
INSTRUMENT DE RESECTION D'HEMORROIDES

(30) Priorität: 02.05.1997 DE 19718708
(43) Veröffentlichungstag der Anmeldung: 15.03.2000
(73) Patentinhaber: Burgard, Gunther, 66424 Homburg (DE)
(72) Erfinder: Burgard, Gunther, 66424 Homburg (DE)
(74) Vertreter: Grünecker, Kinkeldey, Stockmair & Schwanhäusser Anwaltssozietät
(86) Internationale Anmeldenummer: PCT/EP1998/002423
(87) Internationale Veröffentlichungsnummer: WO 1998/049950

(56) Entgegenhaltungen:
- EP-A- 0 688 536
- WO-A-94/07423
- DE-A- 19 522 310
- US-A- 4 428 748
- US-A- 4 998 527
- US-A- 5 084 052
- US-A- 5 234 451

## Beschreibung

Die Erfindung bezieht sich auf ein Hämorrhoidenresektionsinstrument zum minimal-invasiven subanodermalen Entfemen von Hämorrhoidalgewebe.

Das Hämorrhoidalleiden stellt die häufigste Enddarmerkrankung der westlichen zivilisierten Welt dar, seine Erkrankungshäufigkeit wird in der über 50-jährigen Bevölkerung mit 50% angegeben. Hämorrhoiden sind mit Schleimhaut bedeckte Gefäßkonvolute, die sich durch Blutung, Druckgefühl oder Hervortreten bemerkbar machen. Ab einer Hämorrhoidalvergrößerung dritten und vierten Grades ist zur Abhilfe ein operativer Eingriff notwendig, um das Gewebe manuell zu beseitigen.

Hämorrhoidalgewebe bildet sich in der Regel unter dem Anoderm, das heißt unter der Übergangshaut, die sich am Ende des Analkanals zwischen der Schleimhaut des Mastdarms und der außenliegenden Haut der Pobacke befindet. Das Anoderm hat eine extrem hohe Nervendichte, die die sensorische Kontinenz gewährleistet, das ist die Fähigkeit, Gas, Flüssigkeit oder Stuhl zu fühlen, zu unterscheiden und zurückhalten zu können.

Bei den bisher bekannten Verfahren handelt es sich um segmentale Excisionen, z.B. nach Milligan-Morgan, bei denen größere Einschnitte in das hochsensible Anoderm erforderlich sind. Dies kann die Sensorik des Anoderms beeinträchtigen und ist im nachfolgenden Wundheilungsprozeß relativ schmerzhaft für den Patienten.

In der US-A-4,428,748 ist ein Augenoperationsinstrument beschrieben, mit dem Trübungen der Augenlinse operativ behandelt werden können. Der operative Fortsatz dieses Instruments ist nadelartig ausgebildet und hat im Bereich der Spitze eine Fräseröffnung zum Abtragen von Gewebsteilen im Bereich der Augenlinse. Der äußere Teil der Nadel kann ultraschallchirurgisch angetrieben werden. Zum gezielten Abtragen der Bereiche der Augenlinse ist die nadelartige Struktur notwendig, um die abzutragenden Teile im Millimeterbereich gezielt zu behandeln. Für Behandlungen von inhomogenen Gewebestrukturen ist dieses Instrument nicht geeignet.

Die EP 0 591 619 A1 offenbart ein Resektionsinstrument zum operativen Beseitigen von Tumoren und dergleichen zum Beispiel im Bereich von Gallenblasen. Dabei wird unter Anwendung eines Endoskops eine Ultraschalleinheit zum Zerkleinern des Tumors verwendet. Mit Hilfe einer Spülvorrichtung werden die zerkleinerten Tumorpartikel entfernt.

In der US 4,931,047 wird eine Ultraschalleinheit zum Fragmentieren von Tumorgewebe am Verdauungsapparat vorgeschlagen. Mit Hilfe des Ultraschalls wird das Gewebe fragmentiert, um den Tumor zu beseitigen.

In der US 3,945,375 und der US 3,937,222 sind chirurgische Fräsereinheiten offenbart, mit denen Gewebe abgetragen werden kann. Jede Fräsereinheit weist mindestens ein rotierendes Messer auf, das im Bereich einer Öffnung einer Hülse rotiert, so daß im Bereich der Öffnung befindliches Gewebe von dem Fräser abgetragen werden kann.

Der Erfindung liegt die Aufgabe zugrunde, ein Instrument zu schaffen, mit dem eine anathomiegerechte, einfache und schonende operative Entfernung von Hämorrhoidalgewebe möglich ist.

Diese Aufgabe wird erfindungsgemäß gelöst durch ein Resektionsinstrument zum minimal-invasiven, subanodermalen, submucösen Entfernen von Hämorrhoidalgewebe, wobei eine schmale, längliche Trägervorrichtung vorgesehen ist, mit einer vibrationschirurgischen Gewebezerkleinerungseinrichtung und mindestens einer gewebeabtragenden Fräsereinheit.

Zur operativen Anwendung dieses Instruments ist nur ein kleiner Hauteinschnitt, das heißt eine kleine Incision, notwendig. Die Incision wird bevorzugt an der Basis eines Hämorrhoidalknotens am analen Randbereich gemacht. In diesem Bereich endet das Anoderm bzw. geht in die Haut der Pobacke über. Das Anoderm wird dabei nicht oder nur ganz gerinfügig incidiert.

Durch die kleine Incision kann die schmale, längliche Trägervorrichtung unter das Anoderm eingeführt werden. In dem subanodermalen Bereich kann an dem Frontendbereich des Instruments mit der Entfernung des Hämorrhoidalgeflechts begonnen werden.

Mit der Gewebezerkleinerungseinrichtung kann auf vibrationschirurgischem Weg das Hämorrhoidalgewebe präpariert und fragmentiert werden. Es kann vom inneren Schließmuskel auf der einen Seite und vom Anoderm auf der anderen Seite isoliert werden. Dabei bleibt das Anoderm erhalten und der Musculus Intemus unbeschädigt.

Als vibrationschirurgische Einrichtung kann z.B. ein ultraschallchirurgisches Werkzeug verwendet werden, das z.B. im Bereich von 20 000 bis 40 000 Hz betrieben wird. Auf diesem Wege kann das Hämorrhoidalgewebe ganz gezielt und ohne Beschädigung des gesunden Gewebes präpariert und fragmentiert werden.

Anschließend kann mit der gewebeabtragenen Fräsereinheit das präparierte Gewebe zerkleinert und abgetragen werden, so daß es stückweise aus dem Bereich zwischen Schließmuskel und Anoderm entfernbar ist.

Mit diesem Instrument ist eine sehr einfache Operation möglich, die nicht assistenzbedürftig ist und nur ca. ein Drittel der Zeit herkömmlicher Operationsverfahrens benötigt.

Das hochsensible Anoderm bleibt erhalten und kann anschließend anatomiegerecht in den Analkanal zurückverlagert werden. Für den Patienten bedeutet dies, daß weniger gesundes Gewebe zerstört wird, so daß die Wundheilung schneller abläuft. Wegen des erhaltenen Anoderms ist der postoperative Wundschmerz wesentlich geringer.

Vorzugsweise kann die Gewebezerkleinerungseinrichtung in Längsrichtung der Trägervorrichtung gesehen näher an der Spitze der Trägervorrichtung angeordnet sein als die Fräsereinheit. Damit gelangt beim Vorschub des Instruments in das Hämorrhoidalgewebe zuerst die Gewebezerkleinerungseinrichtung in Wirkkontakt mit dem Gewebe. Die Präparation und das Fragmentieren erfolgt in Vorschubrichtung, bevor die Fräsereinheit wirksam wird.

Besonders vorteilhaft kann die Gewebezerkleinerungseinrichtung unmittelbar an der vordersten Spitze der Trägervorrichtung angeordnet sein. Damit kann das Präparieren an dem vordersten Ende der Trägervorrichtung geschehen, so daß die Präparation während des gezielten Vorschubs des Instruments durchgeführt werden kann.

Günstigerweise kann die Fräsereinheit beabstandet von der Spitze der Trägervorrichtung im seitlichen Mantelbereich der Trägervorrichtung angeordnet sein. Damit kann die Fräsereinheit seitlich wirken und setzt in Vorschubrichtung gesehen erst nach der Präparation des Hämorrhoidalgewebes ein.

In besonderer Weise können mindestens zwei voneinander beabstandete Fräseröffnungen der Fräsereinheit im seitlichen Mantelbereich der Trägervorrichtung vorgesehen sein. Damit kann zweiseitig Gewebe entfernt werden, so daß das Hämorrhoidalgewebe relativ rasch und in unterschiedliche räumliche Richtungen hin abgetragen werden kann.

Es wird vorgeschlagen, daß die Fräseröffnungen senkrecht zur Längsachse der Trägervorrichtung gesehen etwa auf derselben Umfangslinie angeordnet sind. Damit ist gewährleistet, daß die Fräswirkung beim Vorschub des Instruments erst ab einer bestimmten Umfangslinie beginnt.

In einer bevorzugten Ausführungsform kann der Frontendbereich eine abgewinkelt zur Längsachse der Trägervorrichtung verlaufende Schrägfläche aufweisen, so daß ein im Querschnitt etwa keilförmiger oder schnabelförmiger Frontendbereich gebildet ist. Diese Form erleichtert den Vorschub des Instruments in dem Gewebe.

Besonders vorteilhaft kann der Schrägungswinkel der Schrägfläche gegenüber der Längsachse der Trägervorrichtung etwa 40° bis 50°, vorzugsweise 45°, betragen. Mit diesem Winkel ist der Frontendbereich noch ausreichend stabil und kann relativ einfach in das Hämorrhoidalgewebe vorgeschoben werden.

Als Variante der Erfindung kann eine Wirkfläche der Gewebezerkleinerungseinrichtung mindestens teilweise in die Schrägfläche integriert sein. Damit kann die Gewebezerkleinerung in einer etwas abgewinkelt zur Vorschubrichtung angeordneten Richtung erfolgen, so daß auch etwas seitlich präpariert werden kann.

Möglicherweise kann am Frontendbereich eine Lichtspendeeinrichtung vorgesehen sein, die diaphanoskopisch verwertbares Licht abgibt. Unter Diaphanoskopie ist das Durchleuchten von Körperteilen zu verstehen, um anhand von Schatten den Aufbau oder die Konturen von Gewebe festzustellen. Beim Gebrauch des Instruments kann der operierende Arzt den Aufbau des Hämorrhoidalgewebes und seine Grenzen sowie umliegendes Gewebe erkennen und das Instrument gezielt an die gewünschte Stelle schieben. Er erkennt krankhaftes Gewebe im Vergleich zu gesundem Gewebe und erkennt die Lage des Frontendes des Instruments, um es gezielt zu den gewünschten Stellen zu führen. Er blickt dabei durch das Anoderm hindurch.

Als Lichtspendeeinrichtung kann z.B. ein Glasfaserlichtleiter zum Frontendbereich führen, der Kaltlicht abgibt.

Vorzugsweise kann die Lichtspendeeinrichtung etwa im Flächenbereich zwischen der Gewebezerkleinerungseinrichtung und der/den Fräsereinheit/en vorgesehen sein. Damit wird der Wirkbereich der Gewebezerkleinerungseinrichtung bzw. der Fräsereinheit beleuchtet und ist vom Chirurgen durch das Gewebe hindurch erkennbar, so daß er das Instrument gezielt steuern kann.

In besonderer Weise kann die Lichtspendeeinrichtung in einer sich abgewinkelt zur Längsachse der Trägervorrichtung erstreckenden Schrägfläche vorgesehen sein. Dies ermöglicht eine etwas schräg abgewinkelte Lichtabstrahlung, so daß der seitlich umliegende Bereich um den Frontendbereich besser angeleuchtet wird und diaphanoskopisch besser erkennbar ist.

Möglicherweise kann die Lichtspendeeinrichtung im Mantelbereich nahe der Fräsereinheit vorgesehen sein. Dadurch wird der mit der Fräsereinheit bearbeitete Arbeitsbereich besonders gut ausgeleuchtet und erkennbar.

Es wird vorgeschlagen, daß die Lichtspendevorrichtung relativ zu den Wirkflächen von Fräsereinheit und/oder Gewebezerkleinerungseinrichtung eine großflächig lichtabgebende Spendefläche hat. Dies sorgt für eine besonders gute Ausleuchtung des zu bearbeitenden Hämorrhoidalgewebes.

Besonders vorteilhaft kann die Trägervorrichtung mindestens einen mit einer Unterdruckpumpe verbindbaren Saugkanal aufweisen, der in eine am Frontendbereich vorgesehene Saugöffnung mündet. Mit Hilfe der Saugöffnung kann im Frontbereich ein leichter Unterdruck erzeugt werden, so daß umliegendes Gewebe leicht an den Frontendbereich gezogen wird. Damit entsteht Kontakt mit der vibrationschirurgischen Gewebezerkleinerungseinrichtung bzw. das abzutragende Gewebe wird der Fräsereinheit zugeführt.

Als Variante der Erfindung kann die Saugöffnung zentral in einer Wirkfläche der Gewebezerkleinerungsvorrichtung vorgesehen sein. So wird das umliegende Gewebe relativ gezielt an die Wirkfläche geführt, so daß das Gewebe beim Kontakt zerkleinert wird.

Denkbar können eine Fräseröffnung/en im Mantelbereich der Trägervorrichtung, in deren Bereich ein in der Trägervorrichtung bewegbares Fräsermesser vorgesehen ist, als Saugöffnung ausgebildet sein. Damit wird Hämorrhoidalgewebe aufgrund leichten Unterdrucks zu dem Fräsermesser gezogen.

In einer bevorzugten Ausführungsform kann die Trägervorrichtung mindestens einen mit einem Spülsystem verbindbaren Spülkanal aufweisen, der in eine im Frontendbereich vorgesehene Spülöffnung mündet. Über die Spülöffnung kann Spülflüssigkeit zugeführt oder abgeführt werden. Dadurch kann der Bereich unter dem Anoderm ausgespült werden und eventuelle freie Gewebeteile gelöst werden. Die Spülflüssigkeit kann entweder durch einen Spülkanal des Instruments wieder zurückfließen oder an der Incisionsstelle zwischen Haut und Instrument austreten. Zum Rückführen von Spülflüssigkeit kann auch der Saugkanal verwendet werden. Prinzipiell kann als Saugkanal und Spülkanal auch derselbe Kanal verwendet werden, in dem er nacheinander in der einen oder anderen Richtung durchströmt wird.

Denkbar kann eine Fräseröffnung im Mantelbereich der Trägervorrichtung, in deren Bereich ein in der Trägervorrichtung bewegbares Fräsermesser vorgesehen ist, als Spülöffnung ausgebildet sein. Damit kann die Spülflüssigkeit auch zum Reinigen der Fräsereinheit benutzt werden, z.B. wenn diese droht, durch Gewebeteile verstopft zu werden. Ferner können Gewebeteile durch die Fräseröffnung weggespült werden. Wahlweise kann die Spülung auch zur Kühlung der Vibrationseinheit dienen.

Vorzugsweise kann die Spülöffnung zentral in einer Wirkfläche der Gewebezerkleinerungseinrichtung vorgesehen sein.

In besonderer Weise kann die Trägervorrichtung ein den Frontendbereich tragendes erstes Teil und ein damit lösbar verbindbares, als Handhabe ausgebildetes zweites Teil aufweisen.

In weiterer Ausgestaltung der Erfindung kann die Handhabe eine die Winkelausrichtung des Frontendbereichs bezüglich der Längsachse der Trägervorrichtung anzeigende Markierung aufweisen. Mit Hilfe der Markierung kann die Drehlage des Frontendbereichs an der außen zugänglichen Handhabe erkannt werden, so daß dadurch die genaue Lage der Gewebezerkleinerungseinrichtung bzw. der Fräsereinheit erkennbar ist.

Möglicherweise kann die Fräsereinheit mit einem in der Trägervorrichtung bewegbaren Fräsermesser versehen sein, das im Bereich einer Fräseröffnung der Trägervorrichtung angeordnet ist. Dabei kann das Fräsermesser z. B. in der Trägervorrichtung rotieren, wobei das Messer im Bereich der Fräseröffnung vorgesehen ist. Gewebe, das sich im Bereich der Fräseröffnung befindet, wird von dem Fräsermesser dosiert zerschnitten und abgetragen. Durch die Anordnung des Fräsermessers in der Trägervorrichtung kann das Fräsermesser relativ geschützt in das Gewebe eingeführt und darin bewegt werden. Nur im Bereich der Fräseröffnung wird das Gewebe abgetragen. Eine derartig ausgebildete Fräsereinheit kann auch als "Shaver" bezeichnet werden.

Gemäß einer bevorzugten Ausführungsform kann die Gewebezerkleinerungseinrichtung und/oder die Fräsereinheit längs verschiebbar in der Trägervorrichtung gelagert sein. Damit kann die Gewebezerkleinerungseinrichtung gezielt zu den zu behandelnden Tumorteilen vorgeschoben werden. Dabei kann die Trägervorrichtung beispielsweise als Hülse oder Trokar ausgebildet sein, in dem die Gewebezerkleinerungseinrichtung von hinten manuell eingeführt wird und über das vordere Ende des Trokars hinausschiebbar ist.

Bevorzugterweise kann die Wirkfläche der Gewebezerkleinerungseinrichtung aus der Trägervorrichtung in eine Stellung hervorschiebbar sein, bei der die Wirkfläche nach vorne aus der Trägervorrichtung hervorragt. Die Wirkfläche kann somit als vorderste Spitze dienen, die sich bereichsweise in Gewebe vorarbeitet und Raum zum Nachführen der Trägervorrichtung schafft. Dadurch ist die Wirkfläche nicht zwangsweise auf die Form des Frontendbereiches der Trägervorrichtung festgelegt, sondern kann separat, z.B. fingerförmig, von der Trägervorrichtung hervorragen.

In besonderer Weise kann die Wirkfläche der Gewebezerkleinerungsvorrichtung etwa ovale oder kreisrunde Form haben.

Ausführungsbeispiele der Erfindung sind in der Zeichnung dargestellt und werden nachstehend erläutert. Es zeigen:
- Fig. 1: eine schematische Seitenansicht eines ersten Teils eines erfindungsgemäßen Instruments gemäß einer ersten Ausführungsform der Erfindung,
- Fig. 2: eine Prinzipskizze der Anordnung von zwei Fräseröffnungen am Frontendbereich eines erfindungsgemäßen Instruments gemäß der ersten Ausführungsform,
- Fig. 3: eine Prinzipskizze der Anordnung der einzelnen Elemente des Frontendbereichs eines erfindungsgemäßen Instruments gemäß der ersten Ausführungsform,
- Fig. 4: eine schematische Skizze einer Handhabe mit anschließbaren Versorgungseinheiten für ein erfindungsgemäßes Instrument gemäß der ersten Ausführungsform,
- Fig. 5: ein Längsschnitt durch den Enddarmbereich mit vergrößertem Hämorrhoidalkomplex,
- Fig. 6: ein Längsschnitt durch einen Enddarmbereich mit entfernten Hämorrhoidalkomplex,
- Fig. 7: ein Längsschnitt durch den Enddarm in postoperativem Zustand,
- Fig. 8: eine schematische Seitenansicht eines ersten Teils eines erfindungsgemäßen Instruments gemäß einer zweiten Ausführungsform der Erfindung und
- Fig. 9: eine Prinzipskizze der Anordnung der einzelnen Elemente des Frontendbereichs eines erfindungsgemäßen Instruments gemäß einer dritten Ausführungsform der Erfindung.

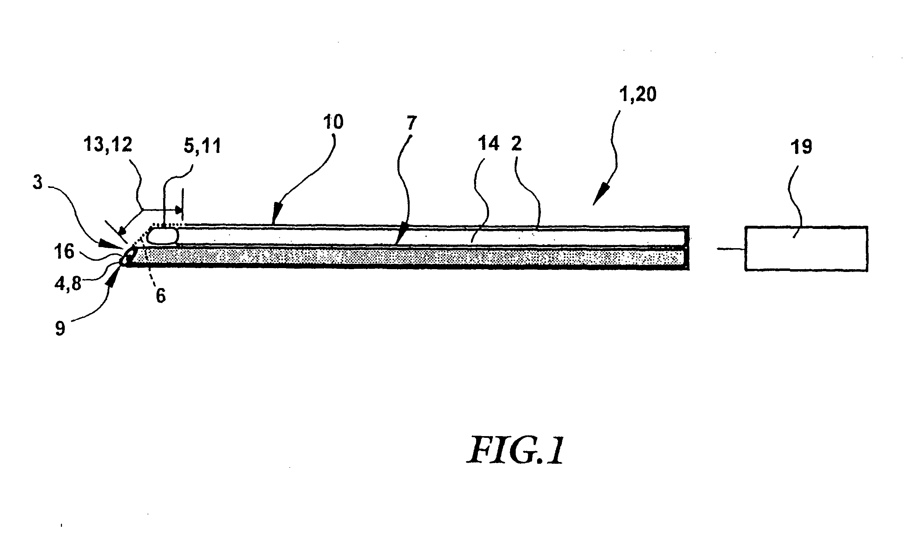

In Figur 1 ist ein erstes Teil 20 eines erfindungsgemäßen Hämorrhoidenresektionsinstruments 1 gemäß einer ersten Ausführungsform schematisch dargestellt. Es umfaßt eine schmale, längliche, im Querschnitt etwa ovale Trägervorrichtung 2. Der Querschnitt ist relativ klein; er kann etwa 0,5 cm betragen.

Das erste Teil 20 ist etwa fingerförmig und hat etwa eine Länge von 7 cm. Die Trägervorrichtung 2 hat ein Frontende 3, an dem eine Gewebezerkleinerungseinrichtung 4 und eine gewebeabtragende Fräsereinheit 5 vorgesehen ist.

Der Frontendbereich hat eine Schrägfläche 6, die gegenüber der Längsachse 7 der Trägervorrichtung etwa um 45° angewinkelt verläuft. In der dargestellten Ansicht bildet der Frontendbereich 3 einen etwa keilförmigen Querschnitt.

In die Schrägfläche 6 ist eine Wirkfläche 8 der Gewebezerkleinerungseinrichtung 4 integriert. Die Wirkfläche 8 hat eine Titanfläche, die in Vibration versetzt werden kann, so daß sie anliegendes Gewebe vibrationschirurgisch zerkleinert. Die Vibration ist vorzugsweise ultraschallchirurgisch mit einem Frequenzbereich von etwa 20 000 bis 40 000 Hz verbunden. Solche Schwingungen werden z.B. durch piezoelektrische Materialien erzeugt, die durch wechselnde Spannung in mechanische Vibration versetzt werden und die Titanfläche zum Schwingen anregen.

Die Wirkfläche 8 befindet sich unmittelbar an der vordersten Spitze 9 der Trägervorrichtung 2. Sie befindet sich in Längsrichtung der Trägervorrichtung gesehen näher an der Spitze 9 als die Fräsereinheit 5.

Die Fräsereinheit 5 ist beabstandet von der Spitze der Trägervorrichtung 2 vorgesehen. Sie weist eine im seitlichen Mantelbereich 10 vorgesehene Fräseröffnung 11 auf. In der Trägervorrichtung ist ein nicht dargestelltes rotierendes Fräsermesser angeordnet, das im Bereich der Fräseröffnung 11 rotiert, so daß durch die Fräseröffnung 11 hindurch Gewebe von dem Fräsermesser abgetragen werden kann.

Eine nicht dargestellte Antriebsachse für das Fräsermesser erstreckt sich längs durch die Trägervorrichtung 2 hindurch bis zu einem entsprechenden externen Gerät. Genauso erstreckt sich die Antriebsmechanik für die Vibration der Gewebezerkleinerungseinrichtung 4 längs durch die Trägervorrichtung 2 zu einem entsprechenden Anschlußgerät hindurch.

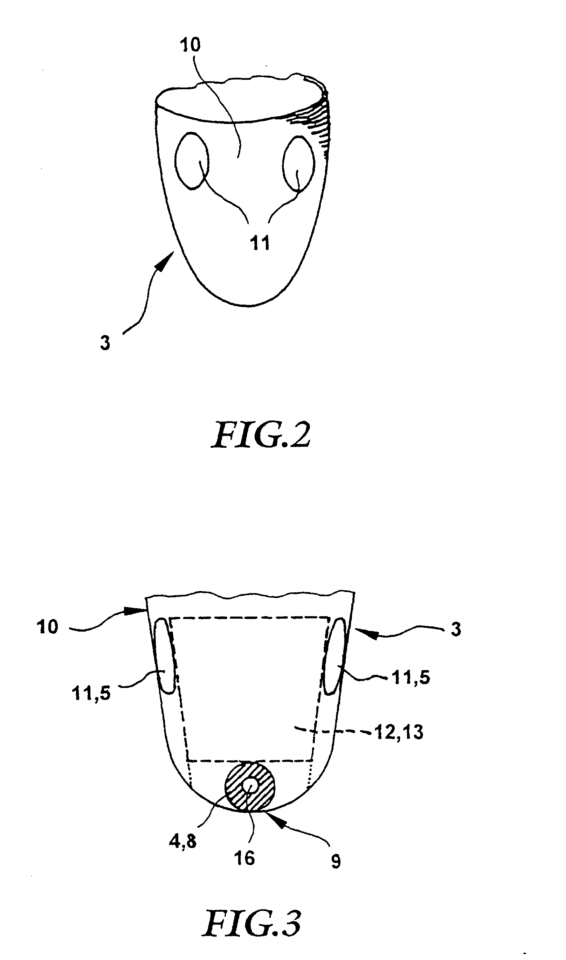

In Figur 2 ist schematisch der Mantelbereich 10 des Frontendbereichs 3 mit zwei Fräseröffnungen 11 dargestellt. Die zwei Fräseröffnungen 11 können wahlweise von demselben rotierenden Messer bedient werden, so daß im Prinzip zwei voneinander beabstandete Fräsereinheiten 5 gebildet sind. Die Fräseröffnungen 11 befinden sich etwa auf derselben Umfangslinie senkrecht zur Längsachse der Trägervorrichtung 2 und sind beabstandet voneinander vorgesehen. Die Fräseröffnungen sind beabstandet zu der Schrägfläche 6 vorgesehen oder grenzen gerade noch an diese an.

Der äußere Mantelbereich 10 kann im senkrechten Querschnitt zur Längsachse gesehen über einen Umfangswinkel von zirka 100 bis 120° keine Fräseröffnungen aufweisen, um in diesem Bereich umliegendes Gewebe nicht zu zerstören.

Wie in den Figuren 1 und 3 dargestellt ist am Frontendbereich eine Lichtspendeeinrichtung 12 vorgesehen. Sie spendet Licht, das zu diaphänoskopischen Betrachtungen verwendbar ist. Diaphanoskopie ist die Durchleuchtung von Gewebeteilen, wobei unterschiedliche Gewebebereiche schattenartig von außen durch das Gewebe hindurch erkennbar sind.

Als Licht kann Kaltlicht verwendet werden, das durch ein sich entlang der Trägervorrichtung 2 erstreckendes Glasfaserkabel transportiert wird.

In Figur 1 ist in punktierter Linie ein lichtspendender Flächenbereich 13 angezeichnet, der im Vergleich zu den Fräseröffnungen 11 und der Wirkfläche 8 großflächig ausgebildet ist. Der Flächenbereich 13 ist teilweise in der Schrägfläche 6 und damit zusammenhängend teilweise in dem Mantelbereich 10 vorgesehen.

In Figur 3 ist schematisch eine gerade Draufsicht auf die Schrägfläche 6 dargestellt. In der Schrägfläche 6 befindet sich der Flächenbereich 13. Im seitlich gerundeten Mantelbereich 10 befinden sich je seitlich eine Fräseröffnung 11. An der Spitze 9 befindet sich die Wirkfläche 8 der Gewebezerkleinerungseinrichtung 4. Der lichtspendende Flächenbereich 13 ist etwa zwischen Wirkfläche 8 und den Fräseröffnungen 11 angeordnet.

Die Fräseröffnungen 11 sind mit einem sich länglich durch die Trägervorrichtung 2 hindurch erstreckenden Saugkanal 14 verbunden, der mit einer Saugpumpe verbindbar ist. Die Fräseröffnung dient somit als Saugöffnung.

Wie in den Figuren 1 und 3 gezeigt, befindet sich zentral in der Wirkfläche eine Saugöffnung 16, die ebenfalls über einen Saugkanal mit der Saugpumpe verbindbar ist.

Die Saugöffnung 16 und die Fräseröffnung 11 können auch wahlweise als Spülöffnung verwendet, wobei der entsprechende Saugkanal dann als Spülkanal verwendbar ist. Wahlweise können auch mehrere separate Spülkanäle vorgesehen sein, so daß z.B. durch die Fräseröffnung 11 Spülflüssigkeit ausgestoßen und durch die Saugöffnung 16 die Spülflüssugkeit wieder eingesaugt werden kann, oder umgekehrt.

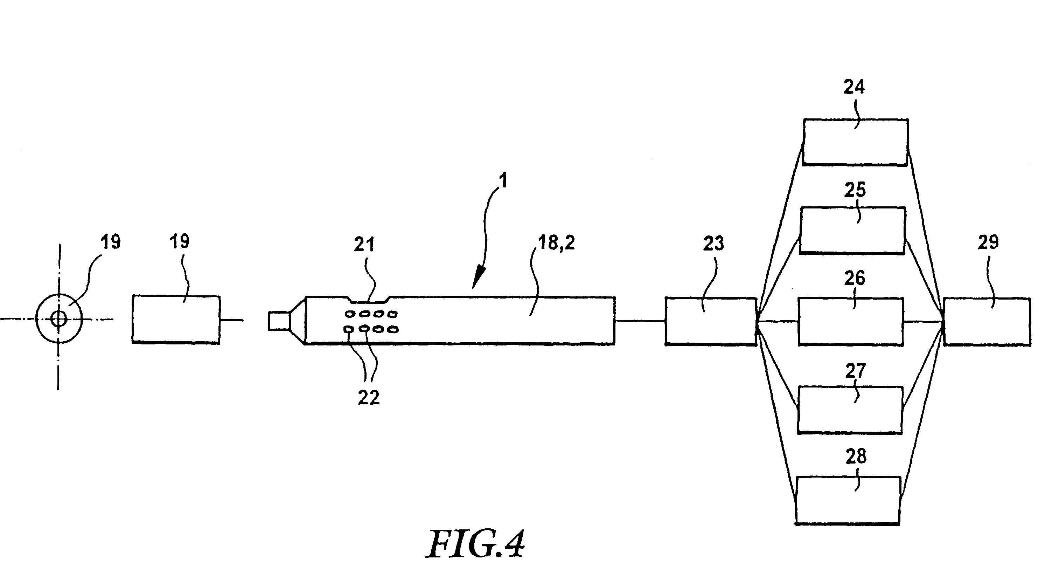

In Figur 4 ist ein zweites Teil 18 der Trägervorrichtung 2 dargestellt, das als Handhabe ausgebildet ist. Die Handhabe 18 ist über ein schematisch dargestelltes Verbindungsglied 19 mit dem ersten Teil 20 lösbar verbindbar. Ebenfalls schematisch ist links ein kreisrunder Hohlquerschnitt des Verbindungsglieds 19 angedeutet.

Die Handhabe 19 weist eine in der Außenseite als Vertiefung eingeformte Markierung 21 auf. Um den Bereich der Markierung 21 sind mehrere Griffnoppen 22 vorgesehen.

Wie schematisch angedeutet, ist das Instrument 1 mit einer Fußschaltung 23 versehen, mit der die Gewebezerkleinerungseinrichtung und/oder die Fräsereinheit unabhängig voneinander zu- und abschaltbar ist. Eventuell können darüber auch die Saugfunktion oder die Spülfunktion geschaltet werden.

Blockartig sind ein Fräserantriebsgerät 24, ein Vibrationsantrieb 25, eine Saugpumpe 26, ein Spülsystem 27 und eine Lichtquelle 28 dargestellt. Alle diese Geräte können durch einen Mikroprozessor 29 geregelt werden.

Das Spülsystem 27 kann auch als Kühlsystem ausgebildet, um das Instrument 1 im Betrieb zu kühlen.

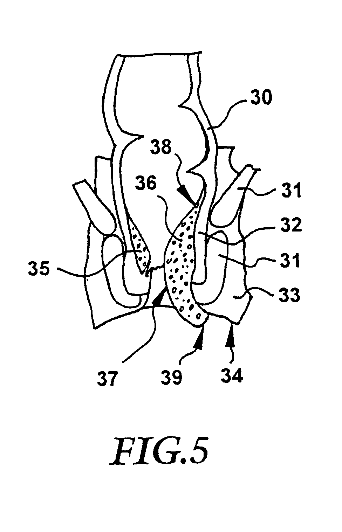

In Figur 5 ist der Enddarmbereich dargestellt, mit dem Enddarm 30, einem äußeren Schließmuskel 31, einem inneren Schließmuskel 32, Unterhautfettgewebe 33 und äußerer Haut 34 einer Pobacke. Nahe der Afteröffnung befinden sich links ein normal großer Hämorrhoidalkomplex 35 und rechts ein vergrößerter Hämorrhoidalkomplex 36, der in der Darstellung etwa einer Vergrößerung vierten Grades entspricht. Das Hämorrhoidalgewebe 36 befindet sich unter dem Anoderm 37, das die Haut zwischen der Haut der Pobacke und der inneren Schleimhaut des Enddarmes bildet. Das Anoderm ist stark mit Nerven durchzogen und sorgt für die sensorische Kontinenz.

Beim Entfernen des Hämorrhoidalgewebes wird zunächst die Blutzufuhr durch Ligaturen auf der inneren Schicht 38 des inneren Schließmuskels 32 unterbunden. Anschließend wird im Basisbereich 39 des Hämorrhoidalgewebes nahe der Haut 34 eine kleine Incision gemacht. Durch diese Incision ist das Instrument 1 in den Komplex 36 subanodermal einführbar. Die Incision kann in der Haut 34 oder auch mindestens bereichsweise am Ansatzbereich des Anoderms 37 gemacht werden.

Es wird zunächst nur die vibrationschirurgische Gewebezerkleinerungseinrichtung 4 benutzt und das Hämorrhoidalgewebe präpariert und fragmentiert. Vorzugsweise wird dabei entlang der Kante des inneren Schließmuskels 32 auf der einen Seite und direkt unterhalb des Anoderms 37 auf der anderen Seite vorgegangen.

Der Weg des Frontendbereichs 3 durch das Gewebe wird dabei mit Hilfe der Lichtspendeeinrichtung 12 diaphanoskopisch von außen durch das Anoderm betrachtet und gesteuert.

Nach dem Präparieren und Fragmentieren wird die Fräsereinheit 4 eingesetzt und das Hämorrhoidalgewebe zerkleinert und mit Hilfe des Spülsystems oder des Saugsystems abgeführt.

Das Saugsystem wird auch dazu eingesetzt, um das Gewebe unter leichtem Unterdruck an die Fräsereinheit 5 oder die Gewebezerkleinerungseinrichtung 4 heranzuführen, damit das Gewebe behandelt werden kann.

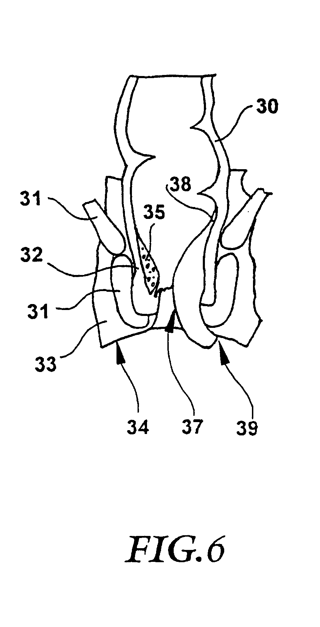

Figur 6 zeigt den Zustand nach kompletter Hämorrhoidalresektion. Sie zeigt den leeren ballonartigen Bereich zwischen Anoderm und innerer Wand des Enddarms.

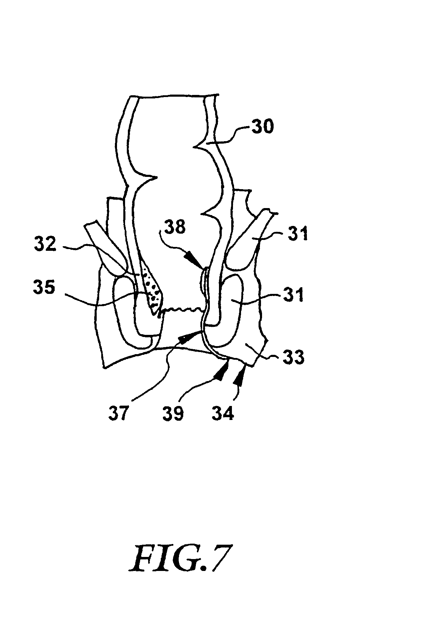

Figur 7 zeigt den postoperativen Zustand, bei dem das Anoderm anatomiegerecht in den Analkanal transpositioniert ist und dort geeignet vernäht ist. Der kleine Einschnitt kann noch zu Drainagezwecken benutzt werden.

Vorteilhafterweise kann das Anoderm während des Eingriffs erhalten bleiben, da das Hämorrhoidalgewebe mit Hilfe des Instruments 1 subanodermal behandelt werden kann.

Das erfindungsgemäße Instrument ermöglicht eine neue Operationstechnik, die als "minimal-invasive subanodermale Hämorrhoidektomie" bezeichnet werden kann. Das Verfahren sowie das Instrument wurde von dem Anmelder selbst entwickelt. Der Einsatz des erfindungsgemäßen Instruments erlaubt erstmalig die subanodermale Resektion des Hämorrhoidalplexus ohne Incision des Anoderm, jedoch mit anatomiegerechter anschließender Transposition desselben in den Analkanal. Das neue Operationsverfahren umfaßt vorzugsweise die folgenden Schritte:
1. Wahlweise subcutanes-submucöses Unterspritzen des Hämorrhoidalknotens, bei zirkulärem Hämorrhoidalprolaps der Hauptsegmente bei 3, 7 und 11 Uhr Steinschnittlage und eventuell der Satellitenknoten mit vasokonstriktorischer Lösung unter Zusatz eines Lokalanästhetikums,
2. Inspektorisches und digitales Aufsuchen (palpable Pulsationen) der Arteria rectalis sup. Äste, Durchstechungsligaturen präliminar transmucös unter Verankerung der Nähte auf der inneren Schicht der Internusmuskulatur, benutzt wird resorbierbares Nahtmaterial Stärke 3 x 0, atraumatisch, Langlassen der Fäden und Anklemmen derselben,
3. Ca. 0,5 bis 1 cm lange Querincision der Haut an der Basis des Hämorrhoidalknotens im Bereich des analen Randsulcus,
4. Freilegung und sichere Identifikation der Kante des Musculus sphincter internus,
5. von diesem Schnitt aus Abpräparieren der Hämorrhoidalgeflechte Richtung Gefäßbasis und zu den seitlichen Segmentgrenzen hin, in der richtigen Schicht, unmittelbar auf der Kante des M. sphincter internus und direkt subanodermal, wobei das erfindungsgemäße Instrument eingesetzt wird, welches mit Hilfe der ultraschallchirurgischen Technik zuerst den Hämorrhoidalplexus vom Internus und vom Anoderm unter diaphanoskopischer Kaltlichtkontrolle isoliert; als weiterer Schritt werden unter dosiertem Sog die entsprechend mobilisierten Geflechte durch Einsatz der Shavertechnik reseziert.

Somit ist das neue Verfahren dadurch gekennzeichnet, daß zum Abpräparieren der Hämorrhoidalgeflechte das erfindungsgemäße Instrument eingesetzt wird, das die Isolierung des Hämorrhoidalplexus vom Internus und vom Anoderm ermöglicht.

Das neue Verfahren unter Einsatz des erfindungsgemäßen Instruments hat folgende Vorteile:
1. Als rekonstruktives Operationsverfahren ist die Technik gegenüber den bekannten Verfahren einfach, nicht assistenzbedürftig, zeitlich weniger aufwendig (ca. ein Drittel der Operationsdauer der Verfahren nach Parks oder Fangler-Arnold).
2. Das hochsensible Anoderm wird primär nicht incidiert, dennoch anatomiegerecht in den Analkanal zurückverlagert, 3 bis 4 kleine Hautincisionen von maximal 1 cm Länge minimieren die resultierende Wundfläche nach "außen".
3. Dadurch deutliche Verminderung der postoperativen Schmerzintensität.
4. Verkürzung der Hospitalisationsdauer, eventuell Durchführung des Eingriffes auf tageschirurgischer Basis.

Die vorliegende Erfindung offenbart somit erstmalig den Einsatz shaverartiger Instrumente, gegebenenfalls in Kombination mit ultraschallchirurgischen Instrumenten, im proktologischen Bereich zur Hämorrhoidenresektion. Wahlweise ist auch der Einsatz des shaverartigen Instruments allein mit einer Diaphanoskopieeinheit denkbar.

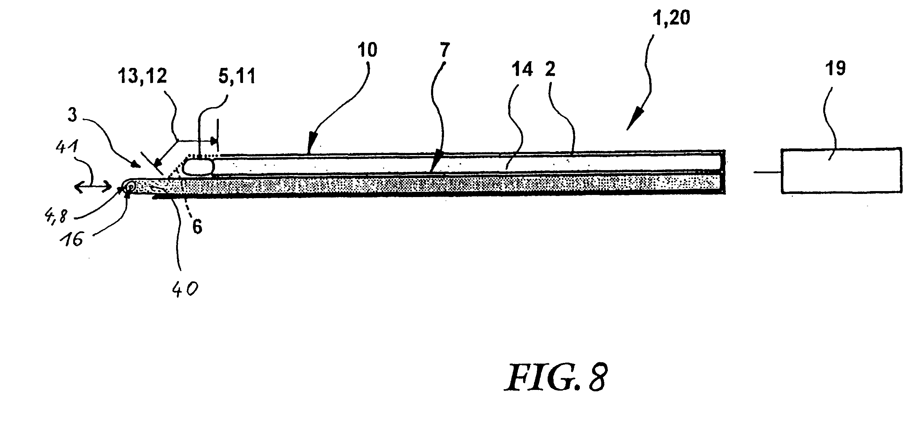

In Figur 8 ist ein erstes Teil 20 eines erfindungsgemäßen Hämorrhoidenresektionsinstruments 1 gemäß einer zweiten Ausführungsform schematisch dargestellt. Die zweite Ausführungsform unterscheidet sich von der ersten Ausführungsform im wesentlichen durch die im folgenden genannten Punkte, so daß im übrigen auf die Beschreibung zur ersten Ausführungsform verwiesen wird.

Die Trägervorrichtung 2 ist etwa hülsenartig bzw. als Trokar ausgebildet, in dem die Fräsereinheit 5 und die Gewebezerkleinerungseinrichtung 4 zusammen oder unabhängig voneinander von einem rückwärtigen Bereich bis in den Frontendbereich 3 einschiebbar sind. Die Gewebezerkleinerungseinrichtung 4 weist einen Stabkörper 40 auf, an dessen Frontende die Wirkfläche 8 angeordnet ist. Die Wirkfläche 8 ist etwa kreisförmig und hat zentral angeordnet eine etwa kreisförmige Saugöffnung 16. Wirkfläche 8 und Saugöffnung 16 sind im Umfangsbereich des Stabkörpers 40 angeordnet. Sie können wahlweise aber auch direkt an der vorderen Spitze vorgesehen sein.

Der Stabkörper 40 ist längs verschiebbar in der Trägervorrichtung 2 angeordnet. Die Verschieberichtung ist mit dem Richtungspfeil 41 angedeutet. In Figur 8 ist eine Stellung dargestellt, bei der die Wirkfläche 8 aus der Trägervorrichtung 2 hervorgeschoben ist und daraus etwa fingerartig hervorragt. Der Stabkörper 40 kann wahlweise auch drehbar in der Trägervorrichtung 2 sein, so daß mit der im Umfangsbereich vorgesehen Wirkfläche 8 allseitig gearbeitet werden kann. Damit der Stabkörper 40 über die Schrägfläche 6 hinaus vorgeschoben werden kann, ist in der Schrägfläche 6 eine geeignete Öffnung vorgesehen.

Im übrigen kann für das zweite erfindungsgemäße Ausführungsbeispiel verständlicherweise auch die Handhabe 18 gemäß der ersten Ausführungsform verwendet werden.

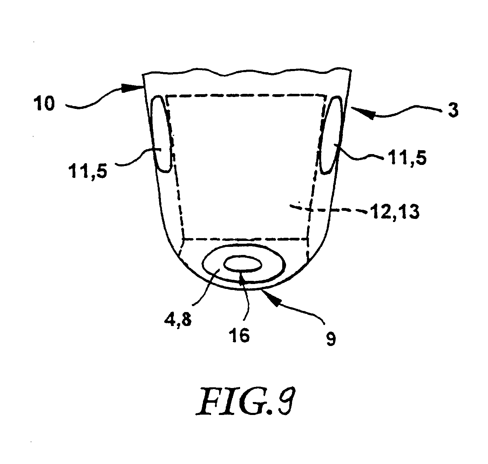

In Figur 9 ist schematisch eine gerade Draufsicht auf die Schrägfläche 6 gemäß einer dritten Ausführungsform der Erfindung dargestellt. Diese Ausführungsform entspricht im wesentlichen der ersten Ausführungsform gemäß Figur 3, so daß auf die diesbezügliche Beschreibung verwiesen wird.

Bei der dritten Ausführungsform ist die Wirkfläche 8 im Vergleich zur ersten und zweiten Ausführungsform oval ausgebildet. Sie kann beispielsweise auch elyptisch ausgebildet sein. Die Saugöffnung 16 ist zentral in der ovalen Form vorgesehen und hat ebenfalls ovale Form. Mit der ovalen Form kann der flächige Bereich der Schrägfläche 6 in Abgrenzung zu dem lichtspendenden Flächenbereich 13 besser ausgenutzt werden, so daß sich eine insgesamt größere Wirkfläche 8 ergibt.

Verständlicherweise kann diese ovale Form auch auf die erste und zweite Ausführungsform der Erfindung übertragen werden.

## Patentansprüche

1. Hämorrhoidenresektionsinstrument (1) zum minimal-invasiven subanodermalen, submucösen Entfernen von Hämorrhoidalgewebe (36), wobei eine schmale, längliche Trägervorrichtung (2) vorgesehen ist, mit einer vibrationschirurgischen Gewebezerkleinerungseinrichtung (4) zum Präparieren und Fragmentieren von Hämorrhoidalgewebe (36) und mindestens einer gewebeabtragenden Fräsereinheit (5).

2. Instrument nach Anspruch 1,
**dadurch gekennzeichnet,**
**daß** die Fräsereinheit (5) ein in der Trägervorrichtung (2) bewegbares Fräsermesser aufweist, welches innerhalb der Trägervorrichtung (2) aufgenommen in das Gewebe einführbar ist.

3. Instrument nach Anspruch 2,
**dadurch gekennzeichnet,**
**daß** die Trägervorrichtung (2) mindestens eine Fräseröffnung (11) aufweist, in deren Bereich das Fräsermesser angeordnet ist.

4. Instrument nach einem der vorhergenden Ansprüche,
**dadurch gekennzeichnet,**
**daß** die Fräsereinheit (5) beabstandet von der Spitze (9) der Trägervorrichtung (2) vorgesehen ist.

5. Instrument nach einem der vorhergenden Ansprüche,
**dadurch gekennzeichnet,**
**daß** mindestens zwei voneinander beabstandete Fräseröffnungen (11) der Fräsereinheit (5) vorgesehen sind.

6. Instrument nach Anspruch 5,
**dadurch gekennzeichnet,**
**daß** die Fräseröffnungen im seitlichen Mantelbereich (10) der Trägervorrichtung vorgesehen sind.

7. Instrument nach Anspruch 5 oder 6,
**dadurch gekennzeichnet,**
**daß** die Fräseröffnungen (11) senkrecht zur Längsachse (7) der Trägervorrichtung (2) gesehen etwa auf derselben Umfangslinie angeordnet sind.

8. Instrument nach einem der vorhergehenden Ansprüche,
**dadurch gekennzeichnet,**
**daß** in einem Frontendbereich (3) der Trägervorrichtung (2) eine Wirkfläche (8) der Gewebezerkleinerungsvorrichtung (4) vorgesehen ist.

9. Instrument nach einem der vorhergehenden Ansprüche,
**dadurch gekennzeichnet,**
**daß** Gewebezerkleinerungseinrichtung (4) und Fräsereinheit (5) an einem Frontendbereich (3) der Trägervorrichtung (2) vorgesehen sind.

10. Instrument nach einem der vorhergehenden Ansprüche,
**dadurch gekennzeichnet,**
**daß** die Gewebezerkleinerungseinrichtung (4) in Längsrichtung (7) der Trägervorrichtung (2) gesehen näher an der Spitze (9) der Trägervorrichtung (2) angeordnet ist als die Fräsereinheit (5).

11. Instrument nach einem der vorhergehenden Ansprüche,
**dadurch gekennzeichnet,**
**daß** die Gewebezerkleinerungseinrichtung (4) unmittelbar an der vordersten Spitze (5) der Trägervorrichtung (2) angeordnet ist.

12. Instrument nach einem der vorhergenden Ansprüche,
**dadurch gekennzeichnet,**
**daß** der Frontendbereich (3) eine abgewinkelt zur Längsachse (7) der Trägervorrichtung (2) verlaufende Schrägfläche (6) aufweist, so daß ein im Querschnitt etwa keilförmiger oder schnabelförmiger Frontendbereich (3) gebildet ist.

13. Instrument nach Anspruch 12,
**dadurch gekennzeichnet,**
**daß** der Schrägungswinkel der Schrägfläche (6) gegenüber der Längsachse (7) der Trägervorrichtung (2) etwa 40° bis 50°, vorzugsweise 45°, beträgt.

14. Instrument nach Anspruch 12 oder 13,
**dadurch gekennzeichnet,**
**daß** eine Wirkfläche (8) der Gewebezerkleinerungseinrichtung (4) mindestens teilweise in die Schrägfläche (6) integriert ist.

15. Instrument nach einem der vorhergenden Ansprüche,
**dadurch gekennzeichnet,**
**daß** eine Lichtspendeeinrichtung (12) vorgesehen ist, die diaphanoskopisch verwertbares Licht abgibt.

16. Instrument nach Anspruch 15,
**dadurch gekennzeichnet,**
**daß** die Lichtspendeeinrichtung (12) etwa im Flächenbereich (13) zwischen der Gewebezerkleinerungseinrichtung (4) und der/den Fräsereinheit/en (5) vorgesehen ist.

17. Instrument nach Anspruch 15 oder 16,
**dadurch gekennzeichnet,**
**daß** die Lichtspendereinrichtung (12) in einer sich abgewinkelt zur Längsachse (7) der Trägervorrichtung (2) erstreckenden Schrägfläche (6) vorgesehen ist.

18. Instrument nach einem der Ansprüche 15 bis 17,
**dadurch gekennzeichnet,**
**daß** die Lichtspendeeinrichtung (12) im Mantelbereich (10) nahe der Fräsereinheit (5) vorgesehen ist.

19. Instrument nach einem der Ansprüche 15 bis 18,
**dadurch gekennzeichnet,**
**daß** die Lichtspendeeinrichtung (12) relativ zu den Wirkflächen von Fräsereinheit (5) und/oder Gewebezerkleinerungseinrichtung (4) eine großflächig lichtabgebende Spendefläche (13) hat.

20. Instrument nach einem der vorhergenden Ansprüche,
**dadurch gekennzeichnet,**
**daß** mindestens ein mit einer Unterdruckpumpe (26) verbindbarer Saugkanal (14) vorgesehen ist, der in eine Saugöffnung (16, 11) der Trägervorrichtung (2) mündet.

21. Instrument nach Anspruch 20,
**dadurch gekennzeichnet,**
**daß** die Saugöffnung (16) zentral in einer Wirkfläche (8) der Gewebezerkleinerungseinrichtung (4) vorgesehen ist.

22. Instrument nach Anspruch 20 oder 21,
**dadurch gekennzeichnet,**
**daß** eine Fräseröffnung/en (11) im Mantelbereich (10) der Trägervorrichtung, in deren Bereich ein in der Trägervorrichtung bewegbares Fräsermesser vorgesehen ist, als Saugöffnung ausgebildet ist.

23. Instrument nach einem der vorhergenden Ansprüche,
**dadurch gekennzeichnet,**
**daß** die Trägervorrichtung (2) mindestens einen mit einem Spülsystem (27) verbindbaren Spülkanal aufweist, der in eine im Frontendbereich (3) vorgesehene Spülöffnung (11, 16) mündet.

24. Instrument nach Anspruch 23,
**dadurch gekennzeichnet,**
**daß** eine Fräseröffnung/en (11) im Mantelbereich der Trägervorrichtung, in deren Bereich ein in der Trägervorrichtung bewegbares Fräsermesser vorgesehen ist, als Spülöffnung ausgebildet ist.

25. Instrument nach Anspruch 23 oder 24,
**dadurch gekennzeichnet,**
**daß** die Spülöffnung (16) zentral in einer Wirkfläche (8) der Gewebezerkleinerungsvorrichtung (4) vorgesehen ist.

26. Instrument nach einem der vorhergenden Ansprüche,
**dadurch gekennzeichnet,**
**daß** die Trägervorrichtung (2) ein den Frontendbereich tragendes erstes Teil (20) und ein damit lösbar verbindbares, als Handhabe (18) ausgebildetes zweites Teil (18) aufweist.

27. Instrument nach einem der vorhergenden Ansprüche,
**dadurch gekennzeichnet,**
**daß** eine Handhabe (18) der Trägervorrichtung (2) eine die Winkelausrichtung des Frontendbereichs (3) bezüglich der Längsachse (7) der Trägervorrichtung (2) anzeigende Markierung (29) aufweist.

28. Instrument nach einem der vorhergehenden Ansprüche,
**dadurch gekennzeichnet,**
**daß** Gewebezerkleinerungseinrichtung (4) und Fräsereinheit (5) unabhängig voneinander betätigbar sind.

29. Instrument nach einem der vorhergehenden Ansprüche,
**dadurch gekennzeichnet,**
**daß** die Trägervorrichtung (2) einen Hohlquerschnitt aufweist, durch den die Fräsereinheit (5) und die Gewebezerkleinerungseinrichtung (4) zuführbar sind.

30. Instrument nach einem der vorhergehenden Ansprüche,
**dadurch gekennzeichnet,**
**daß** die Gewebezerkleinerungseinrichtung (4) und/oder die Fräsereinheit längs verschiebbar in der Trägervorrichtung gelagert ist.

31. Instrument nach einem der vorhergehenden Ansprüche,
**dadurch gekennzeichnet,**
**daß** die Wirkfläche der Gewebezerkleinerungseinrichtung aus der Trägervorrichtung in eine Stellung hervorschiebbar ist, bei der die Wirkfläche nach vorne aus der Trägervorrichtung hervorragt.

32. Instrument nach einem der vorhergehenden Ansprüche,
**dadurch gekennzeichnet,**
**daß** die Wirkfläche der Gewebezerkleinerungsvorrichtung etwa ovale oder kreisrunde Form hat.

## Revendications

1. Instrument de résection d'hémorroïdes (1) pour le retrait sous-cutané anal et sous-muqueux mini-invasif de tissu hémorroïdal (36), dans lequel un dispositif porteur étroit allongé (2) est prévu, avec un équipement chirurgical vibratoire de broyage de tissu (4) pour la préparation et la fragmentation de tissu hémorroïdal (36) et au moins une unité de fraise (5) pour aplanir le tissu.

2. Instrument selon la revendication 1, **caractérisé en ce que** l'unité de fraise (5) présente dans le dispositif porteur (2) une lame de fraise mobile qui, incluse dans le dispositif porteur (2), peut être introduite dans le tissu.

3. Instrument selon la revendication 2, **caractérisé en ce que** le dispositif porteur (2) présente au moins une ouverture de fraise (11) dans la région de laquelle la lame de fraise est placée.

4. Instrument selon l'une quelconque des revendications précédentes, **caractérisé en ce que** l'unité de fraise (5) est placée en position décalée par rapport à la pointe (9) du dispositif porteur (2).

5. Instrument selon l'une quelconque des revendications précédentes, **caractérisé en ce qu'**au moins deux ouvertures de fraise (11) décalées l'une par rapport à l'autre sont placées sur l'unité de fraise (5).

6. Instrument selon la revendication 5, **caractérisé en ce que** les ouvertures de fraise sont placées dans la zone d'enveloppe (10) du dispositif porteur.

7. Instrument selon la revendication 5 ou 6, **caractérisé en ce que** les ouvertures de fraise (11) sont placées perpendiculairement à l'axe longitudinal (7) du dispositif porteur (2) à peu près sur la même ligne en périphérie.

8. Instrument selon l'une quelconque des revendications précédentes, **caractérisé en ce que** dans la zone d'extrémité avant (3) du dispositif porteur (2) est placée une surface active (8) de l'équipement de broyage du tissu (4).

9. Instrument selon l'une quelconque des revendications précédentes, **caractérisé en ce que** l'équipement de broyage du tissu (4) et l'unité de fraise (5) sont placés sur une zone d'extrémité avant (3) du dispositif porteur (2).

10. Instrument selon l'une quelconque des revendications précédentes, **caractérisé en ce que** l'équipement de broyage du tissu (4), en direction longitudinale (7) du dispositif porteur (2), est placé plus près de la pointe (9) du dispositif porteur (2) que l'unité de fraise (5).

11. Instrument selon l'une quelconque des revendications précédentes, **caractérisé en ce que** l'équipement de broyage du tissu (4) est placé immédiatement à la pointe la plus en avant (5) du dispositif porteur (2).

12. Instrument selon l'une quelconque des revendications précédentes, **caractérisé en ce que** la zone d'extrémité avant (3) présente une surface oblique (6) en angle par rapport à l'axe longitudinal (7) du dispositif porteur (2) de sorte que soit formée en coupe une zone d'extrémité avant (3) à peu près en forme de coin ou en forme de bec.

13. Instrument selon la revendication 12, **caractérisé en ce que** l'angle d'inclinaison de la surface oblique (6) par rapport à l'axe longitudinal (7) du dispositif porteur (2) est de 40° à 50°, de préférence de 45°.

14. Instrument selon la revendication 12 ou 13, **caractérisé en ce qu'**une surface active (8) de l'équipement de broyage du tissu (4) est intégrée au moins en partie dans la surface oblique (6).

15. Instrument selon l'une quelconque des revendications précédentes, **caractérisé en ce qu'**un dispositif d'éclairage (12) est prévu qui fournit une lumière utilisable en diaphanoscopie.

16. Instrument selon la revendication 15, **caractérisé en ce que** le dispositif d'éclairage (12) est placé dans la zone de surface (13) entre l'équipement de broyage du tissu (4) et la ou les unités de fraise (5).

17. Instrument selon la revendication 15 ou 16, **caractérisé en ce que** le dispositif d'éclairage (12) est placé sur une surface oblique (6) s'étendant de manière inclinée par rapport à l'axe longitudinal (7) du dispositif porteur (2).

18. Instrument selon l'une des revendications 15 à 17, **caractérisé en ce que** le dispositif d'éclairage (12) est placé dans la zone d'enveloppe (10) à proximité de l'unité de fraise (5).

19. Instrument selon l'une des revendications 15 à 18, **caractérisé en ce que** le dispositif d'éclairage (12) par rapport à la surface effective de l'unité de fraise (5) et/ou de l'équipement de broyage du tissu (4) a une surface dispensatrice d'éclairage (13) étendue.

20. Instrument selon l'une quelconque des revendications précédentes, **caractérisé en ce qu'**au moins un canal d'aspiration (14) pouvant être relié à une pompe à dépression (26) est prévu qui débouche sur une ouverture d'aspiration (16, 11) du dispositif porteur (2).

21. Instrument selon la revendication 20, **caractérisé en ce que** l'ouverture d'aspiration (16) est placée en position centrale dans une surface active (8) de l'équipement de broyage du tissu (4).

22. Instrument selon la revendication 20 ou 21, **caractérisé en ce qu'**une ou des ouvertures de fraise (11) dans la zone d'enveloppe (10) du dispositif porteur, dans la région de laquelle une lame de fraise mobile est placée dans le dispositif porteur, sont formées comme ouvertures d'aspiration.

23. Instrument selon l'une quelconque des revendications précédentes, **caractérisé en ce que** le dispositif porteur (2) présente au moins un canal de lavage pouvant être relié à un système de lavage (27) qui débouche sur une ouverture de lavage (11, 16) prévue dans une zone d'extrémité avant (3).

24. Instrument selon la revendication 23, **caractérisé en ce qu'**une ou des ouvertures de fraise (11) dans la zone d'enveloppe du dispositif porteur, dans la région de laquelle une lame de fraise mobile est placée dans le dispositif porteur, sont formées comme ouvertures de lavage.

25. Instrument selon la revendication 23 ou 24, **caractérisé en ce que** l'ouverture de lavage (16) est placée en position centrale dans une surface active (8) de l'équipement de broyage du tissu (4).

26. Instrument selon l'une quelconque des revendications précédentes, **caractérisé en ce que** le dispositif porteur (2) présente une première partie (20) portant la zone d'extrémité avant et une seconde partie (18) pouvant y être reliée de manière amovible formée comme poignée (18).

27. Instrument selon l'une quelconque des revendications précédentes, **caractérisé en ce qu'**une poignée (18) du dispositif porteur (2) présente un marquage (29) montrant l'orientation de l'angle de la zone d'extrémité avant (3) par rapport à l'axe longitudinal (7) du dispositif porteur (2).

28. Instrument selon l'une quelconque des revendications précédentes, **caractérisé en ce que** l'équipement de broyage du tissu (4) et l'unité de fraise (5) peuvent être actionnés indépendamment l'un de l'autre.

29. Instrument selon l'une quelconque des revendications précédentes, **caractérisé en ce que** le dispositif porteur (2) présente une section creuse à travers laquelle l'unité de fraise (5) et l'équipement de broyage du tissu (4) peuvent être introduits.

30. Instrument selon l'une quelconque des revendications précédentes, **caractérisé en ce que** l'équipement de broyage du tissu (4) et/ou l'unité de fraise sont placés dans le dispositif porteur de manière à pouvoir être déplacés longitudinalement.

31. Instrument selon l'une quelconque des revendications précédentes, **caractérisé en ce que** la surface active de l'équipement de broyage du tissu peut être déplacée pour sortir du dispositif porteur en une position où la surface active dépasse du dispositif porteur vers l'avant.

32. Instrument selon l'une quelconque des revendications précédentes, **caractérisé en ce que** la surface active de l'équipement de broyage du tissu a une forme à peu près ovale ou circulaire.

## Claims

1. An instrument (1) for resectioning hemorrhoids for the minimal invasive subanodermal removal of hemorrhoidal tissue (36), with a narrow, elongated carrier device (2) being provided, comprising a vibrating surgical tissue size-reducing device (4) for the preparation and fragmentation of hemorrhoidal tissue (36), and at least one tissue-removing milling unit (5).

2. The instrument according to claim 1,
**characterized in**
**that** the milling unit (5) comprises a milling knife which is movable in the carrier device (2) and which, being accommodated within the carrier device (2), is insertable into the tissue.

3. The instrument according to claim 2,
**characterized in**
**that** the carrier device (2) comprises at least one milling opening (11) in the area of which the milling knife is arranged.

4. The instrument according to any one of the preceding claims,
**characterized in**
**that** the milling unit (5) is spaced apart from the tip (9) of the carrier device (2).

5. The instrument according to any one of the preceding claims,
**characterized in**
**that** at least two spaced-apart milling openings (11) of the milling unit (5) are provided.

6. The instrument according to claim 5,
**characterized in**
**that** the milling openings are provided in the lateral circumferential area (10) of the carrier device.

7. The instrument according to claim 5 or 6,
**characterized in**
**that** the milling openings (11) are arranged approximately on the same circumferential line when viewed in a direction perpendicular to the longitudinal axis (7) of the carrier device (2).

8. The instrument according to any one of the preceding claims,
**characterized in**
**that** an active surface (8) of the tissue size-reducing device (4) is provided in a front end portion (3) of the carrier device (2).

9. The instrument according to any one of the preceding claims,
**characterized in**
**that** the tissue size-reducing device (4) and the milling unit (5) are provided on a front end portion (3) of the carrier device (2).

10. The instrument according to any one of the preceding claims,
**characterized in**
**that** when viewed in the longitudinal direction (7) of the carrier device (2) the tissue size-reducing device (4) is arranged closer to the tip (9) of the carrier device (2) than the milling unit (5).

11. The instrument according to any one of the preceding claims,
**characterized in**
**that** the tissue size-reducing device (4) is arranged directly at the foremost tip (5) of the carrier device (2).

12. The instrument according to any one of the preceding claims,
**characterized in**
**that** the front end portion (3) has an inclined surface (6) extending at an angle relative to the longitudinal axis (7) of the carrier device (2), resulting in the formation of a front end portion (3) having an approximately wedge-shaped or beak-shaped cross-section.

13. The instrument according to claim 12,
**characterized in**
**that** the angle of inclination of the inclined surface (6) relative to the longitudinal axis (7) of the carrier device (2) is about 40° to 50°, preferably 45°.

14. The instrument according to claim 12 or 13,
**characterized in**
**that** an active surface (8) of the tissue size-reducing device (4) is at least partially integrated into the inclined surface (6).

15. The instrument according to any one of the preceding claims,
**characterized in**
**that** a light providing device (12) is provided for emitting diaphanoscopically usable light.

16. The instrument according to claim 15,
**characterized in**
**that** the light providing device (12) is disposed approximately in the surface area (13) between the tissue size-reducing device (4) and the milling unit(s) (5).

17. The instrument according to claim 15 or 16,
**characterized in**
**that** the light providing device (12) is disposed in an inclined surface (6) extending at an angle relative to the longitudinal axis (7) of the carrier device (2).

18. The instrument according to any one of claims 15 to 17,
**characterized in**
**that** the light providing device (12) is provided in the circumferential area (10) near the milling unit (5).

19. The instrument according to any one of claims 15 to 18,
**characterized in**
**that** the light providing device (12) has a large-surface light-emitting surface (13) relative to the active surfaces of milling unit (5) and/or tissue size-reducing device (4).

20. The instrument according to any one of the preceding claims,
**characterized in**
**that** there is provided at least one suction channel (14) which is connectable to a vacuum pump (26) and terminates in a suction opening (16, 11) of the carrier device (2).

21. The instrument according to claim 20,
**characterized in**
**that** the suction opening (16) is centrally provided in an active surface (8) of the tissue size-reducing device (4).

22. The instrument according to claim 20 or 21,
**characterized in**
**that** a milling opening/milling openings (11) is/are formed as a suction opening in the circumferential area (10) of the carrier device, with a milling knife that is movable in the carrier device being provided in the area thereof.

23. The instrument according to any one of the preceding claims,
**characterized in**
**that** the carrier device (2) comprises at least one flushing channel which is connectable to a flushing system (27) and terminates in a flushing opening (11, 16) provided in the front end portion (3).

24. The instrument according to claim 23,
**characterized in**
**that** a milling opening/milling openings (11) is/are formed as a flushing opening in the circumferential area of the carrier device, with a milling knife that is movable in the carrier device being provided in the area thereof.

25. The instrument according to claim 23 or 24,
**characterized in**
**that** the flushing opening (26) is centrally provided in an active surface (8) of the tissue size-reducing device (4).

26. The instrument according to any one of the preceding claims,
**characterized in**
**that** the carrier device (2) comprises a first part (20) carrying the front end portion and a second part (18) which is detachably connectable to said first part and designed as a handle (18).

27. The instrument according to any one of the preceding claims,
**characterized in**
**that** a handle (18) of the carrier device (2) has a marking (29) indicative of the angular orientation of the front end portion (3) relative to the longitudinal axis (7) of the carrier device (2).

28. The instrument according to any one of the preceding claims,
**characterized in**
**that** the tissue size-reducing device (4) and the milling unit (5) are operable independently of each other.

29. The instrument according to any one of the preceding claims,
**characterized in**
**that** the carrier device (8) has a hollow cross-section through which the milling unit (5) and the tissue size-reducing device (4) can be supplied.

30. The instrument according to any one of the preceding claims,
**characterized in**
**that** the tissue size-reducing device (4) and/or the milling unit is/are supported in the carrier device to be longitudinally displaceable.

31. The instrument according to any one of the preceding claims,
**characterized in**
**that** the active surface of the tissue size-reducing device can be pushed out of the carrier device into a position in which the active surface projects forwards from the carrier device.

32. The instrument according to any one of the preceding claims,
**characterized in**
**that** the active surface of the tissue size-reducing device has an approximately oval or circular shape.
